# EUROPEAN PATENT APPLICATION

(11) **EP 4 706 648 A1**
(43) Date of publication of application: **11.03.2026**
(21) Application number: 24306446.6
(22) Date of filing: 05.09.2024
(51) Int. Cl.: A61K 31/137, A61K 31/335, A61P 9/00, A61P 9/02, A61P 9/04, A61P 37/08, A61K 9/00

(54) **FORMULATIONS OF ADRENALIN AND ANTIDEPRESSANT**

(71) Applicant: BIOPROJET, 75003 Paris (FR)
(72) Inventor: DUVAUCHELLE, Thierry, 94420 LE PLESSIS-TREVISE (FR); ROBERT, Philippe, 35740 PACÉ (FR); LIGNEAU, Xavier, 35760 SAINT GREGOIRE (FR); LANDAIS, Laurent, 35340 ERCÉ-PRÈS-LIFFRÉ (FR); SCHWARTZ, Jean-Charles, 75014 PARIS (FR); LECOMTE, Jeanne-Marie, 75003 PARIS (FR)
(74) Representative: Lavoix

(57) **Abstract**

The present invention concerns formulations of adrenalin with an antidepressant and N-acetylcysteine or a derivative thereof, providing higher exposure to adrenalin, and exhibiting improved stability, and their use for the treatment of shocks.

## Description

The present invention concerns novel pharmaceutical compositions for use in the treatment of shocks.

Circulatory shocks, commonly known as "shocks", are life-threatening medical emergencies wherein the organs and tissues of the body are not receiving an adequate flow of blood and thus an adequate level of oxygen. There are three major types of shocks: cardiogenic, hypovolemic, and distributive shocks. Among distributive shocks, the anaphylactic shock and septic shock can be cited.

Among the symptoms of shocks, it can be cited tachycardia, hypotension and signs of poor end-organ perfusion such as low urine output, confusion or weakness.

Among these shocks, the anaphylactic shock can be cited. Anaphylaxis is a severe allergic reaction of rapid onset affecting many body systems and may cause death. It is due to the release of inflammatory mediators and cytokines from mast cells and basophils, typically due to an immunologic reaction but also, sometimes, non-immunologic mechanisms.

In the immunologic mechanism, immunoglobulin E (IgE) binds to an antigen. Antigen-bound IgE then activates FcεRI receptors (Fc epsilon RI receptors) on mast cells and basophils. This leads to release of inflammatory mediators such as histamine. These mediators subsequently increase the contraction of bronchial smooth muscles, trigger vasodilation, increase the leakage of fluid from blood vessels, and cause heart muscle depression.

Non-immunologic mechanisms involve substances that directly cause the degranulation of mast cells and basophils. These include agents such as contrast media, penicillins, opioids, temperature (hot or cold), and vibration.

The prevalence of severe anaphylaxis is high and dramatically increasing each year. For instance, in France, a recent publication (Monneret-Vautrin, Rev. Fr. Allerg. Immunol. Clin. 2008, 48, 171) quotes a prevalence of 1/10,000 inhabitants and a mortality of 1 per million inhabitants, thereby illustrating the seriousness of the problem.

The balance of evidence from human observations and animal studies suggests that the main pathophysiologic features of anaphylactic shock are a profound reduction in venous tone and fluid extravasation causing reduced venous return (mixed hypovolemic-distributive shock) and depressed myocardial function.

In the occurrence of shocks and more particularly of anaphylactic reactions, an injection of adrenalin (also called epinephrine or adrenalin) within minutes of the onset of symptoms can be lifesaving (Kemp SF et al. Allergy, 2008, 63, 1061-1070). Administration of adrenalin will increase vascular tone, myocardial contractility, and cardiac output in most cases. Adrenalin is a well-known emergency treatment of circulatory shocks such as anaphylactic shock, cardiac arrest, and cardiovascular distress associated with anaphylactic shock, haemorrhagic shock, traumatic shock, infectious shock and secondary shock due to cardiac surgery.

For the treatment of shocks, auto-injectable formulations of adrenalin are commercially available (Anapen^{®}, Epipen^{®}). Adrenalin is typically formulated with sulfites, such as sodium metabisulfite (SMBS) in an aqueous solution for intramuscular injection at a dose of 0.15 mg, 0.3 mg or 0.5 mg (150 µg, 300 µg or 500 µg of adrenalin in 0.3 mL of solution, respectively).

WO2014/053579 discloses the use of a combination of adrenalin and an antidepressant for the treatment of shocks. These combinations were shown to provide an accelerated bioavailability of adrenalin.

MBS are primarily marketed in the form of sodium metabisulfite (SMBS, E223) or potassium metabisulfite (KMBS, E224). When dissolved in water, these salts release the hydrogen sulfite HSO⁻₃ anion to exert their antioxidant activity, which is herein believed to protect adrenalin from oxidative degradation.

However, sulfites are implicated is asthmatic reactions and may also cause symptoms in non-asthmatic individuals, namely dermatitis, urticaria, flushing, hypotension, abdominal pain and diarrhea, and even life-threatening anaphylaxis.

Additionally, this treatment is not always successful. Therefore, there is a need to provide a new and improved treatment of shocks and more particularly of the anaphylactic shock.

As shocks are a life-threatening condition, there is a constant need to improve the rapidity of onset and/or the overall exposure to adrenalin for the patient. It is also desirable to improve the shelf-life of the pharmaceutical compositions.

It has now been discovered that the pharmacokinetics, pharmacodynamics and/or stability of formulations of adrenalin, including combinations of adrenalin and antidepressant, could be improved when formulated with N-acetylcysteine or a derivative thereof.

Surprisingly, the present inventors discovered that the combination of adrenalin together with N-acetylcysteine and derivatives thereof lead to a higher titer of adrenalin.

Without being bound by theory, it is herein believed that N-acetylcysteine and derivatives thereof are able to protect adrenalin from oxidation, thus unexpectedly leading improved diffusion of adrenalin into the general circulation, leading to a rapid and sustained plasma level, and/or increased exposure to adrenalin.

According to a first object, the present invention concerns a combination comprising
adrenalin;
an antidepressant; and
N-acetylcysteine or a derivative thereof.

More specifically, the present invention concerns the above combination for use in the treatment of shocks, for which the emergency treatment typically comprises the administration of adrenalin.

The combination according to the present invention was shown to potentiate the onset and/or action of adrenalin, thus involving a synergy between its ingredients. Such effects are of great interest as they can be life-saving.

The invention therefore allows the combination of a stable, ready-to-use treatment of shocks with an improved bioavailability in particular in the first ten minutes following the intramuscular or subcutaneous injection of the combination adrenalin-antidepressant.

The combination according to the invention is thus of great interest in shocks for which the usual treatment requires the quick administration of adrenalin as it improves the bioavailability of adrenalin.

It is thus an object of the present invention to provide a new use for the treatment for shocks, such as anaphylactic shocks in which the bioavailability of adrenalin is improved.

### Adrenalin

Adrenalin (or epinephrine) is both a hormone and a neurotransmitter. It belongs to the group of catecholamines. Adrenalin as used herein refers to the formula: 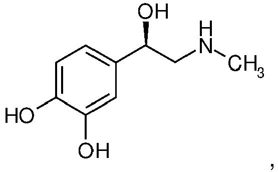 as well as its pharmaceutically acceptable salts.

### N-acetylcysteine

N-acetylcysteine (NAC) is of formula:

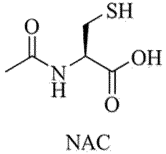

It is routinely used in paracetamol intoxication and as a mucolytic agent.

NAC derivatives are known, such as those described in Med.Chem.Comm. 2017 Dec 1; 8(12): 2238-2247. NAC derivatives include in particular N-acetylcysteine amide (NACA) of formula:

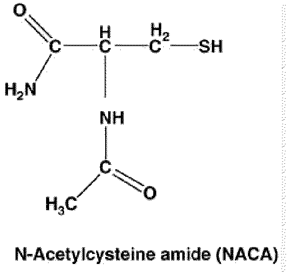

### The antidepressant

The term « antidepressant » refers to an active principle which is used for the prevention and/or treatment of depression. Depression is a mood disorder characterized by an all-encompassing low mood accompanied by low self-esteem, and by loss of interest or pleasure in normally enjoyable activities.

In one embodiment, the antidepressant is an inhibitor of noradrenalin/monoamine transporters as well as an antagonist of the alpha-1 adrenergic receptors and an antagonist of the H1 receptors.

By "antagonist of the H1 receptor" is meant a compound generally having a Ki inferior to 35 nM for the H1-receptors. By "antagonist of the alpha-1 adrenergic receptor" is meant a compound having a Ki inferior to 200 nM for the alpha-1 adrenergic receptors. By "inhibitor of the noradrenalin/monoamine transporter" is meant a compound having a Ki inferior to 100 nM for the noradrenalin/monoamine transporters.

By "Ki" is meant the dissociation constant obtained by inhibiting the binding of a ligand to a target (e.g., receptor) or the inhibition constant obtained by inhibiting noradrenalin/adrenalin uptake.

The antidepressant can be chosen among the following classes:
- Serotonin-norepinephrine reuptake inhibitors (SNRls),
- Serotonin antagonist and reuptake inhibitors (SARIs),
- Norepinephrine reuptake inhibitors (NRls),
- Norepinephrine-dopamine reuptake inhibitors (NDRIs),
- Norepinephrine-dopamine releasing agents (NDRAs),
- Tricyclic antidepressants (TCAs), and
- Tetracyclic antidepressants (TeCAs).

In one embodiment, the antidepressant is chosen among the tricyclic antidepressants (TCAs) or one of their pharmaceutically acceptable salts.

TCAs include in particular:
amitriptyline, amoxapine, amitriptylinoxide, butriptyline, clomipramine, demexiptiline, desipramine, dibenzepin, dimetacrine, dosulepin/dothiepin, doxepin, imipramine, imipraminoxide, lofepramine, maprotiline, mianserin, melitracen, metapramine, nitroxazepine, nortriptyline, noxiptiline, pipofezine, propizepine, protriptyline, quinupramine, amineptine (norepinephrine-dopamine reuptake inhibitor), and trimipramine.

In one embodiment, TCAs include:
amitriptyline, amoxapine, amitriptylinoxide, butriptyline, clomipramine, demexiptiline, dibenzepin, dimetacrine, dosulepin/dothiepin, doxepin, imipraminoxide, lofepramine, maprotiline, mianserin, melitracen, metapramine, nitroxazepine, nortriptyline, noxiptiline, pipofezine, propizepine, protriptyline, quinupramine, amineptine (norepinephrine-dopamine reuptake inhibitor), and trimipramine, or one of their pharmaceutically acceptable salts.

In another embodiment, the antidepressant is chosen among the group consisting of amitryptyline, amoxapine, clomipramine, doxepin, imipramine, maprotiline, mianserin nortriptyline, protriptyline and trimipramine. More preferably the antidepressant is chosen among the group consisting of doxepin, trimipramine, amitriptyline and mianserin. More preferably, the antidepressant is chosen among doxepin or one of its pharmaceutically acceptable salts, preferably doxepin hydrochloride, imipramine or one of its pharmaceutically acceptable salts, preferably imipramine hydrochloride or amitryptiline or one of its pharmaceutically acceptable salts, preferably amitryptiline hydrochloride.

According to an embodiment, said antidepressant is doxepin or one of its pharmaceutically acceptable salts.

### The combinations

The combinations are herein to be understood as actual combined preparations or as kits-of-parts i.e. as separate components which are meant to be combined, such as for combined administration.

According to an embodiment, said combination may be formulated in the form of a single aqueous solution, or alternatively as a kit, in the form of two aqueous solutions.

According to an embodiment, when said combination is in the form of a combined preparation i.e. a single formulation, it comprises N-acetylcysteine or a derivative thereof as the sole antioxidant i.e. it does not comprise alternative oxidant, in particular it is devoid of metabisulfite (MBS) such as sodium metabisulfite (SMBS). According to an embodiment, said combination does not comprise MBS, such as SMBS.

According to said alternative form, said kit may comprise a first formulation comprising adrenalin and be N-acetylcysteine or a derivative thereof on the one hand, and a second formulation comprising the antidepressant on the other hand.

According to an embodiment said first formulation may comprises one or more additional antioxidant such as ascorbic acid or a bisulfite derivative such as sodium metabisulfite (SMBS). According to a preferred embodiment, it comprises SMBS.

According to an embodiment, said second formulation may comprise an antioxidant or may be devoid of an antioxidant. When it is present, the antioxidant may be chosen from N-acetylcysteine and derivatives and ascorbic acid. Preferably, said second formulation is devoid of bisulfite derivative, in particular it is devoid of sodium metabisulfite (SMBS).

### The pharmaceutically acceptable salts

The term "pharmaceutically acceptable salts" refers to salts which retain the biological effectiveness and properties of the active ingredient, and which are not biologically or otherwise undesirable. Pharmaceutically acceptable acid addition salts may be prepared from inorganic and organic acids, while pharmaceutically acceptable base addition salts can be prepared from inorganic and organic bases. For a review of pharmaceutically acceptable salts see Berge et al. ((1977) J. Pharm. Sd, vol. 66, 1). For example, the salts include those derived from inorganic acids such as hydrochloric, hydrobromic, sulfuric, sulfamic, phosphoric, nitric, and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, fumaric, methanesulfonic, and toluenesulfonic acid and the like.

The preferred pharmaceutically acceptable salts for the antidepressant are chosen among hydrochloride, mesylate, maleate and fumarate.

The preferred pharmaceutically acceptable salts for doxepin are doxepin hydrochloride salts.

The preferred pharmaceutically acceptable salts for adrenalin are hydrochloride and tartrate salt.

The present invention also encompasses hydrates or hydrated salts or polymorphic crystalline structures, racemates, diastereomers or enantiomers of the above ingredients.

### Pharmaceutical compositions

According to a further object, the present invention also provides a pharmaceutical composition comprising the combination according to the invention, together with one or more pharmaceutically acceptable excipients.

According to an embodiment, the pharmaceutical composition may further comprise one or more additional ingredients typically chosen from pharmaceutically acceptable excipients, such as preservative agents, buffers, substances to make the composition isotonic with blood such as sodium chloride, solvents, stabilizers, or antimicrobial preservatives. Pharmaceutically acceptable excipients are well-known to the skilled person. They should not adversely affect the stability, bioavailability, safety, or efficacy of the active ingredients, or cause toxicity or undue local irritation when the composition is administered.

Suitable pharmaceutically excipients for the present invention include in particular salts including sodium chloride, complexing agents such as EDTA, EGTA, alkaline or acidic agents such as NaOH and HCl, respectively.

According to a preferred embodiment, the composition may comprise adrenalin, doxepin, N-acetylcysteine, EDTA, EGTA, sodium chloride and water.

According to a still preferred embodiment, said combination is an aqueous solution consisting of adrenalin, doxepin, N-acetylcysteine, EDTA, EGTA, sodium chloride and water.

According to a still preferred embodiment, the pH of said combination typically to a pH comprised between 1 and 7, preferably between 2 and 5, more preferably between 2.5 and 3.5, to ensure the stability of the combination. The pH can be adjusted using appropriate buffers, and/or acid or alkaline agents, such HCl or NaOH.

In an embodiment, the use of the pharmaceutical composition as defined above, comprises administering a dose of adrenalin comprised between 0.01 mg and 12 mg, preferably comprised between 0.05 mg and 10 mg. Preferably, the use of the pharmaceutical composition as defined above, comprises administering a dose of adrenalin comprised between 0.05 mg and 0.15 mg for children and between 0.1 mg and 1 mg, preferably between 0.15 mg and 1 mg, more preferably between 0.3 and 0.5 mg for adults. More preferably, the use of the pharmaceutical composition as defined above comprises administering a dose of adrenalin of 0.1 mg for children and of 0.3 mg for adults.

In another embodiment, the pharmaceutical composition for use as defined above, comprises administering the antidepressant at a dose comprised between 0.02 mg and 1200 mg, preferably between 0.05 mg and 200 mg.

### Administration routes

The pharmaceutical compositions as defined above may be suitable for various administration routes including the oral, sublingual, sub-cutaneous, intramuscular, intravenous, topical, local, intratracheal, intranasal, transdermal or rectal administration.

In a preferred embodiment, the invention relates to a pharmaceutical composition suitable for injection for example via intramuscular route.

An injection is an instrumental method used to introduce into the body a liquid pharmaceutical composition by parenteral administration.

By injection is preferably meant a method of administration which can be intramuscular, subcutaneous or a transcutaneous penetration. By transcutaneous penetration is to be understood by local pressure without skin perforation by needle. More preferably, the pharmaceutical composition of the invention is suitable for intramuscular injection and/or subcutaneous injection.

According to an embodiment, the pharmaceutical composition as defined above may be administered by intramuscular injection. Alternative routes of administration, such as transcutaneous administration including intranasal, sublingual or buccal administration may be contemplated.

In one embodiment, the pharmaceutical composition of the invention is in a unit dosage form in which the volume to be injected is comprised between 0.025 ml and 0.5 ml. More preferably, the injected volume is of 0.05 to 0.3 ml.

In one embodiment, in the solution for injection, adrenalin is at a concentration comprised between 0.05 mg/ml and 80.0 mg/ml. In a particular embodiment, adrenalin is at a concentration between 0.15 mg/ml and 20.0 mg/ml. In another embodiment, adrenalin is at a concentration between 0.2 mg/ml and 20 mg/ml in formulations suitable for adults. In another embodiment, adrenalin is at a concentration between 0.1 mg/ml and 6 mg/ml in formulations suitable for children.

In another embodiment, in the solution for injection, the antidepressant is at a concentration comprised between 0.05 mg/ml and 800 mg/ml. Preferably, the antidepressant is at a concentration comprised between 0.1 mg/ml and 60 mg/ml.

In a pharmaceutical composition suitable for intramuscular injection, the ratio (in concentration) of adrenalin to the antidepressant (i.e., adrenalin concentration/ doxepin concentration) may be typically comprised between 1/1 and 1/10.

The pharmaceutical composition or combination as defined above may also be suitable for intranasal administration, i.e. suitable to be delivered into the nasal cavity of a patient.

The administration route typically depends on the form of said composition or combination.

When it is in the form of a liquid formulation, such as an aqueous solution or suspension, it may be sprayed into the nasal cavity of the patient.

The term "spray" refers to a liquid formulation that is blown or driven through the air in the form of tiny drops. Typically, it refers to a liquid that may be kept in a can or other container, and may be forced out in very small drops.

When it is in the form of a liquid formulation, the volume to be administered is typically comprised between 0.025 ml and 0.50 ml.

In one embodiment, in a liquid formulation to be sprayed, adrenalin is typically at a concentration comprised between 0.5 mg/ml and 100 mg/ml, preferably between 1 mg/ml and 80 mg/ml. In a particular embodiment, adrenalin is at a concentration between 1.5 mg/ml and 20.0 mg/ml. In another embodiment, adrenalin is at a concentration between 2 mg/ml and 10 mg/ml in formulations suitable for adults. In another embodiment, adrenalin is at a concentration between 1 mg/ml and 30 mg/ml in formulations suitable for children.

In an embodiment, in the liquid formulation to be sprayed as defined above, the antidepressant is at a concentration comprised between 0.5 mg/ml and 2000 mg/ml. Preferably, the antidepressant is at a concentration comprised between 1.5 mg/ml and 50 mg/ml.

When the pharmaceutical composition is in a solid form such as a dry-powder, the composition may be administered by inhalation and delivered with a dry-powder inhaler.

When it is in the form of a dry-powder, the total weight to be administered is comprised between 0.05 and 60 mg.

In an embodiment, in a dry-powder formulation, adrenalin is typically at a dose comprised between 0.01 mg and 12 mg. In a particular embodiment, adrenalin is at a dose between 0.15 mg and 0.6 mg, more preferably between 0.2 mg and 0.6 mg in formulations suitable for adults, or between 0.1 mg and 0.3 mg in formulations suitable for children.

In another embodiment, in a dry-powder formulation, said antidepressant is at a concentration comprised between 0.05 mg and 1200 mg, preferably between 0.15 mg and 12 mg.

According to an embodiment, in a pharmaceutical composition suitable for intra-nasal administration according to the invention, the ratio (in concentration) of adrenalin to the antidepressant (i.e., adrenaline concentration/ antidepressant concentration) may be typically comprised between 1/1 and 1/20, preferably between 1/3 and 1/10.

In an embodiment, the combination is formulated in a unit dosage form for single use.

In another embodiment, the combination is formulated in a dosage form for multiple administrations, including multiple successive administrations repeated or sequenced over time.

### Shocks

The invention also relates to the combination or pharmaceutical composition as defined above, for use in the treatment of shocks.

By "shock" it is understood a circulatory shock, which may be characterized by a decrease of the organ perfusion. Circulatory shocks are acute and severe pathologies, often life-threatening, and are well-known by physicians.

The term "shock" refers to any shock as defined herein, for which the emergency treatment typically comprises the administration of adrenalin.

In one particular embodiment, the shock is chosen from the group consisting of: anaphylactic shock, cardiac arrest, and cardiovascular distress associated with anaphylactic shock, haemorrhagic shock, traumatic shock, infectious shock and secondary shock due to cardiac surgery. In a particular embodiment, the shock is the anaphylactic shock.

By "treatment", it may be understood the treatment of the causes of the shock and/or the treatment of its symptoms, more particularly the treatment of its symptoms.

The invention also relates to the combination or pharmaceutical composition as defined above, for use in the treatment of shocks.

The invention also relates to a method of treatment of shocks, such as anaphylactic shocks, comprising the administration of a pharmaceutical composition as defined above in a patient in need thereof.

The present invention also encompasses the combination or pharmaceutical composition of the invention formulated as one single aqueous solution, or two separate aqueous solutions as defined above for use for the treatment of shocks for simultaneous administration.

### The devices

In an embodiment, the dosage form is contained in a self-administration device.

The invention thus also relates to a self-administration device comprising the combination or the pharmaceutical composition of the invention.

According to an embodiment, said self-administration device may be for intramuscular injection, including auto-injection device.

Auto-injection devices also called autoinjectors are medical devices designed to deliver a single dose of a pharmaceutical composition. They are easy and ready to use and are intended for self-administration by patients, or administration by untrained personnel. The site of injection depends on the pharmaceutical composition, but it typically is administered into the thigh or the buttocks. Auto-injection devices for adrenalin are well-known by a man skilled in the art such as Anapen^{®}, Epipen^{®}, Twinject^{®}, Intelliject^{®} or Crossject^{®}. They allow the administration of injectable solutions by intramuscular injection or subcutaneous injection.

In one embodiment, the self-administration device comprises a container prefilled with the pharmaceutical composition as defined above. Preferably, said container is a prefilled syringe in case of injection or a pre-filled spraying device for nasal administration.

In an embodiment, the self-administration device comprises two containers wherein the first container is prefilled with said first formulation as defined above, and the second container is prefilled with said second formulation as defined above, wherein the pharmaceutical composition of the invention is formed by mixing the first and second solutions within the device.

This particular embodiment allows when performing the administration, the administration of the two formulations at the same time and at the same point of injection.

In one embodiment, the first formulation of adrenalin and N-acetylcysteine or a derivative thereof, and the second formulation of antidepressant may be separate, provided they are co-administered at the same time, and at the same point of injection.

By "same point of injection" is understood the area of action of the antidepressant which is the area where the antidepressant blocks totally or partially the local vasoconstriction induced by injected adrenalin, therefore accelerating the systemic bioavailability and potentiates the activity of adrenalin.

By "same time" is meant the time of action of the antidepressant which is the time it takes for the antidepressant to block totally or partially the local vasoconstriction induced by injected adrenalin, therefore accelerating the systemic bioavailability and potentiating the activity of adrenalin

In a particular embodiment, the auto-injection device as defined above comprises at least one prefilled container which is (are) suitable for injection of a volume of the pharmaceutical composition as defined above comprised between 0.05 ml and 0.5 ml. Preferably, the injected volume of the pharmaceutical composition of the invention is of 0.1 to 0.3 ml.

According to an alternative embodiment, said self-administration device may be for intranasal spraying or inhalation. Accordingly, self-administration devices of the invention may include spraying devices and dry powder inhalers.

According to an embodiment, said self-administration device may be suitable for intra-nasal spraying of a liquid formulation. Accordingly, self-administration devices of the invention include spraying devices.

Alternatively, said self-administration device may be suitable for intra-nasal inhalation of a dry-powder formulation. Accordingly, self-administration devices of the invention may include dry-powder inhalers.

In one embodiment, the self-administration device comprises a vial prefilled with the pharmaceutical composition as defined above. Said vial may be a glass vial prefilled with the pharmaceutical composition as defined above, where said glass vial may be further inserted within an opaque container.

In another embodiment, the self-administration device comprises two vials wherein one first vial is prefilled with a first formulation as defined above, the second vial is prefilled with a second formulation as defined above, and wherein the pharmaceutical composition of the invention is formed by mixing the first and second formulations within the device upon delivery.

This particular embodiment allows the mixing and combined administration of the two formulations at the same time upon delivery.

Typically, this embodiment also allows extemporaneous preparation of a liquid formulation when one the first or second formulation is in the form of a powder and the other formulation is in the form of a liquid formulation.

Typically, the powder formulation is preferred for the less stable ingredient or ingredient that is poorly stable in an aqueous formulation.

The following examples are presented as particular embodiments of the invention and cannot be considered as a limitation to the present invention.

### FIGURES

Figure 1 illustrates the comparison of adrenalin concentration following administration by intramuscular route of compositions of the invention (compositions 2, 3 and 4) compared to commercial Anapen^{®} 150 and 300 formulations (compositions 1 and 5, respectively).
Figure 2 illustrates the comparison of adrenalin concentration following administration by intramuscular route of compositions of the invention (compositions D and E) compared to commercial Anapen^{®} 300 and 500 formulations (compositions A and B, respectively).
Figure 3 illustrates the comparison of adrenalin concentration following administration by intramuscular route of compositions of the invention (composition 3) compared to commercial Anapen^{®} 300 and 500 formulations (compositions 1 and 2, respectively).

### EXAMPLES

The increased efficacy and/or bioavailability of the combination of the invention was evidenced in several in vitro and/or animal tests, some of which are described in the following examples.

### 1. Phase 1 trials

Phase 1 studies were conducted on human at various doses of adrenalin and doxepin to study the variation of adrenalin concentration over time following administration. The tested formulations were administered by intramuscular route.

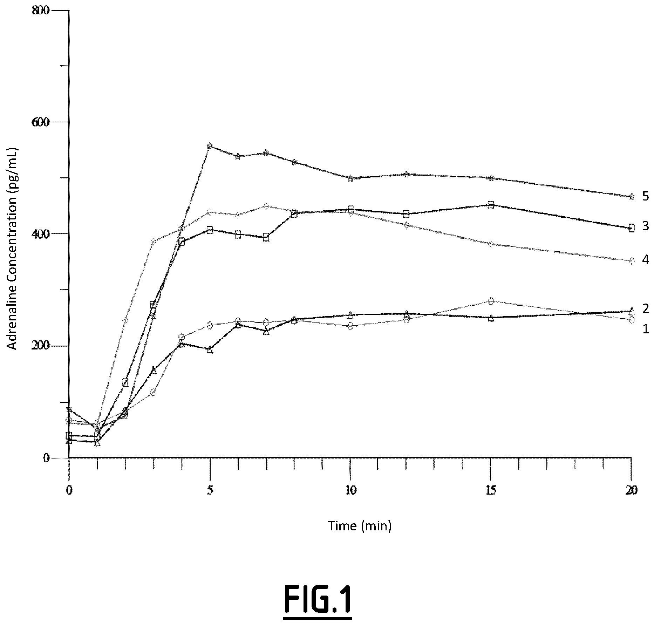

From these Phase 1 studies, 3 sets of data are presented in Figures 1, 2 and 3 showing the plasma adrenalin levels based on different formulations, as represented in Figure 1:
Composition 1: (Adrenalin 150µg + SMBS 1.7 mg/ml)/0.3 ml, illustrative of commercial Anapen^{®} 150
Composition 2: (Adrenalin 150µg + Doxepin 150µg + NAC 1 g/L)/0.3 ml in an Anapen^{®} device, illustrative of the invention
Composition 3: (Adrenalin 150µg + Doxepin 300µg + NAC 1 g/L)/0.3 ml in an Anapen^{®} device, illustrative of the invention
Composition 4: (Adrenalin 150µg + Doxepin 600µg + NAC 1 g/L)/0.3 ml in an Anapen^{®} device, illustrative of the invention
Composition 5: Adrenalin 300µg with 0.51 mg SMBS, illustrative of commercial Anapen^{®} 300

Results are illustrated in Figure 1 which shows that the combination (Adrenalin 150µg + Doxepin 600µg + NAC)/0.3 ml in an Anapen^{®} device (composition 4) provided the earliest increase in adrenalin plasma levels with a maximal effect significantly higher to the one reached with adrenalin 150µg alone (composition 1) and rather like the one reached with adrenalin 300µg alone (composition 5).

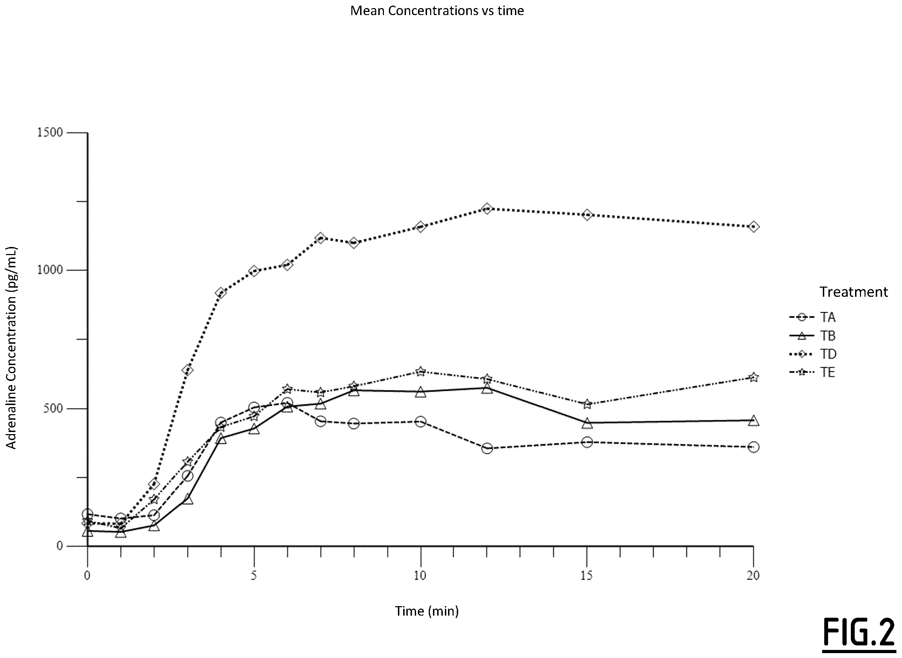

### Additional results are illustrated in Figure 2 with the following compositions:

Composition A: (Adrenalin 300µg + SMBS 1.7 mg/ml)/0.3 ml, illustrative of commercial Anapen^{®} 300
Composition B: (Adrenalin 500µg + SMBS 1.7 mg/ml)/0.3 ml, illustrative of commercial Anapen^{®} 500
Composition D: (Adrenalin 300µg + Doxepin 600µg + NAC 1 g/L)/0.3 ml in an Anapen^{®} device, illustrative of the invention
Composition E: (Adrenalin 150µg + Doxepin 600µg + NAC 1 g/L)/0.3 ml in an Anapen^{®} device, illustrative of the invention

Results are illustrated in Figure 2 which shows the combination (Adrenalin 300µg + Doxepin 600µg + NAC)/0.3 ml in an Anapen^{®} device (composition D) provided the earliest increase in adrenalin plasma levels with a maximal effect significantly higher to those reached with commercial adrenalin 300µg or 500µg alone (compositions A and B). As already shown in Figure 1, the combination (Adrenalin 150µg + Doxepin 600µg + NAC)/0.3 ml in an Anapen^{®} device (composition E) elicited a maximal effect like the one elicited by commercial adrenalin alone at 300µg (composition A).

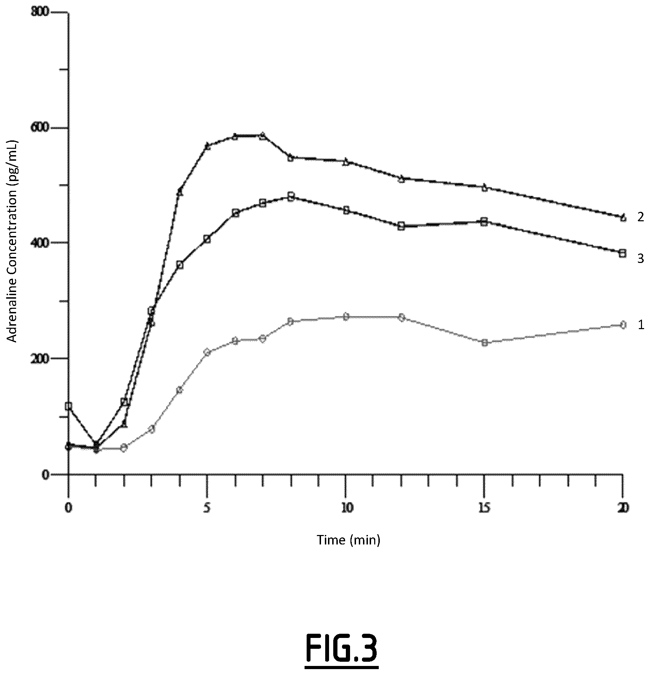

### Figure 3 illustrates results collected with the following compositions:

Composition 1: (Adrenalin 300µg + SMBS 1.7 mg/ml)/0.3 ml, illustrative of commercial Anapen^{®} 300
Composition 2: (Adrenalin 500µg + SMBS 1.7 mg/ml /0.3 ml, illustrative of commercial Anapen^{®} 500
Composition 3: (Adrenalin 300µg + Doxepin 900µg + NAC 1 g/L)/0.3 ml in an Anapen^{®} device, illustrative of the invention

Results are illustrated in Figure 3 which shows that the combination (Adrenalin 300µg + Doxepin 900µg + NAC)/0.3 ml (composition 3) increased earlier adrenalin plasma levels and significantly its maximal plasma levels compared to adrenalin 300µg alone (composition 1). The increase rate in adrenalin plasma levels is like the one recorded with commercial adrenalin 500µg alone (composition 2) and the maximal increase in the same range.

### 2. Stability studies

2.1 Stability over time of formulations comprising both adrenalin (1 mg/ml) and doxepin (1 mg/ml) and either SMBS (1.95 mg/mL) or NAC (0.93 mg/ml) was compared.

Results below were collected following 1 week of storage at 40°C:

| | Adrenalin | Doxepin |
|---|---|---|
| | % Bias versus initial concentration | |
| SMBS | -2,5 | -16,4 |
| NAC | 0,0 | -2,4 |

These data show that both adrenalin and doxepin are more stable when formulated with NAC than when formulated with SMBS.

2.2 Long-term stability over time of formulations comprising both adrenalin (1 mg/ml) and doxepin (1 mg/ml) and either SMBS (1.95 mg/ml), ascorbic acid (0.93 mg/ml) and or NAC (0.93 mg/ml) was compared.

Results below were collected following 3 months of storage at 40°C:

| | Adrenalin | Doxepin |
|---|---|---|
| | % Bias versus initial concentration | |
| SMBS | -35,8 | -27,7 |
| Ascorbic acid | -9,6 | -16,1 |
| NAC | -11,8 | -10,2 |

These data show that both adrenalin and doxepin are more stable over long periods when formulated with NAC than when formulated with SMBS, or ascorbic acid.

These results also show that a preserving agent may be efficient for one of the active ingredient, but not necessarily both. N-acetylcysteine surprisingly improves stability of both doxepin and adrenalin.

## Claims

1. A combination comprising
adrenalin;
an antidepressant, wherein the antidepressant is chosen from the group consisting of amitriptyline, amoxapine, amitriptylinoxide, butriptyline, clomipramine, demexiptiline, dibenzepin, dimetacrine, dosulepin, doxepin, imipraminoxide, lofepramine, maprotiline, mianserin, melitracen, metapramine, nitroxazepine, nortriptyline, noxiptiline, pipofezine, propizepine, protriptyline, quinupramine, amineptine and trimipramine, or one of its pharmaceutically acceptable salts; and
N-acetylcysteine or a derivative thereof.

2. The combination according to claim 1, wherein the antidepressant is doxepin or one of its pharmaceutically acceptable salts.

3. A pharmaceutical composition comprising the combination according to any one of claims 1 to 2, together with one or more pharmaceutically acceptable excipients.

4. The pharmaceutical composition according to claim 3, which is an aqueous solution comprising adrenalin, doxepin, N-acetylcysteine, EDTA, EGTA, sodium chloride, HCl, NaOH and water.

5. The pharmaceutical composition according to claim 3 or 4, which is suitable for intramuscular injection.

6. The pharmaceutical composition according to claim 5 wherein the ratio (in concentration) of adrenalin to the antidepressant (adrenalin dose/ doxepin dose) is comprised between 1/1 and 1/10.

7. The pharmaceutical composition according to claim 4, which is suitable for intranasal administration.

8. The pharmaceutical composition according to claim 7 wherein the ratio (in concentration) of adrenalin to the antidepressant (adrenalin dose/ doxepin dose) is comprised between 1/1 and 1/20.

9. The combination according to anyone of claims 1 to 2 or the pharmaceutical composition according to anyone of claims 3 to 8 for use in the treatment of shocks.

10. The combination for use or the pharmaceutical composition for use according to claim 9 where the shock is selected from the group consisting of: anaphylactic shock, cardiac arrest, and cardiovascular distress associated with anaphylactic shock, haemorrhagic shock, traumatic shock, infectious shock and secondary shock due to cardiac surgery.

11. A self-administration device comprising the combination according to anyone of claims 1 to 2 or the pharmaceutical composition according to anyone of claims 3 to 8.

12. The self-administration device according to claim 11 which is for intramuscular injection or for intranasal administration.

13. The self-administration device according to claim 11 or 12 which comprises a container prefilled with the combination according to anyone of claims 1 to 2 or the pharmaceutical composition according to anyone of claims 3 to 8.

14. The self-administration device according to claim 11 or 12 comprising two containers wherein a first container is prefilled with a first formulaton comprising adrenalin and N-acetylcysteine or a derivative thereof, a second container is prefilled with a second formulation comprising said antidepressant, wherein the pharmaceutical composition according to any one of claims 3 to 8 is formed by mixing the first and second formulations within the device.

15. The self-administration device according to claim 14 wherein one of first formulation or the second formulation is in the form of a powder and the other one or second formulation is in the form of a liquid formulation, and wherein the pharmaceutical composition in the form of a liquid formulation is extemporaneous prepared formed by mixing the first and second formulations within the device.
